# EUROPEAN PATENT APPLICATION

(11) **EP 4 046 660 A1**
(43) Date of publication of application: **24.08.2022**
(21) Application number: 20876446.4
(22) Date of filing: 14.10.2020
(51) Int. Cl.: A61L 2/03, A61L 2/24, A61N 1/40

(54) **DEVICE DESIGNED TO GENERATE AN ELECTRICAL CURRENT IN A CONDUCTOR, METHOD FOR GENERATING AN ELECTRICAL CURRENT IN A CONDUCTOR, METHOD FOR REMOVING BIOFILM FROM A CONDUCTOR INSIDE A PATIENT, AND METHOD FOR TREATING AN INDIVIDUAL REQUIRING REMOVAL OF BIOFILM FROM A METAL PROSTHESIS IMPLANTED IN THEIR BODY**

(30) Priority: 14.10.2019 BR 102019021568
(71) Applicant: Moreira Pacca, Pablo, 05436-020 São Paulo - SP (BR); Vivian Pinto Pacca, Paulo, 04550-000 São Paulo - SP (BR)
(72) Inventor: MOREIRA PACCA, Daniel, 05409-010 São Paulo - SP (BR)
(74) Representative: Uexküll & Stolberg
(86) International application number: PCT/BR2020/050409
(87) International publication number: WO 2021/072519

(57) **Abstract**

The present invention refers to a device and a method to generate an electric current in a conductor (15).

More specifically, the present invention refers to a device (10) that comprises a magnetic field generator element (11), a control element (12) and a support element (14), wherein the magnetic field generator element (11) and the control element (12) are electrically associated to each other, the magnetic field generator element (11) is set to generate a variable magnetic field (B), said variable magnetic field (B) comprises at least one variable parameter, the control element (12) is set to control the at least one variable parameter and the support element (14) is set to hold the magnetic field generator element (11) and the control element.

## Description

The present invention refers to a device able to induce an electric current in a conductor, and to a method to generate an electric current in a conductor by using such device.

### Background

The world population has been constantly increasing, as well as its access to health assistance. So, we are experiencing a demographic transition characterized by a growing proportion of elderly people.

Therefore, the currently expressive number of individuals using medical implants is expected to increase, and there is unfortunately an estimate of a growing number of concerns resulting from implants, such as the occurrence of infections. In the United States, there are 1 million infection cases per year, with an estimated treatment cost of 50 thousand Dollars per subject.

In addition to the relevant costs, a well-known concern discussed by the state of art refers to biofilm formation in such implants. Biofilm can adhere to several materials, such as plastic and metallic materials, Teflon, etc. In metallic implants, the biofilm formation prevents a suitable penetration of antibiotics, thus giving rise to the need of surgical treatments, hospital admissions, open surgeries, extended antibiotic therapy, removal and/or replacement of prosthesis, and long rehabilitation periods with high impact on morbidity and mortality rates, as well as on the economic and social fields.

The above-mentioned biofilm consists in structured communities of bacteria surrounded by polymeric matrices produced by themselves. Such polymeric substances provide protection to the bacteria against various types of possible aggressions, such as the use of any antibiotic or chemical agent to the elimination thereof.

A possible approach to manage these concerns lies on using electricity to reduce the biofilm colony formed in the implants.

The Patent Application published in the United States under # US 2015/0076000 discloses a method to reduce or prevent the growth of micro-organisms in the surface of a medical implant, wherein such method comprises the use of a counter electrode and of a reference electrode. The implant is taken as a working electrode. The electric current traverses the working electrode and the counter electrode. The current passing through the counter electrode is varied in such a way that the electric potential of the working electrode is constant in relation to the electric potential of the reference electrode.

However, the method described by said document does not remedy one of the major inconveniences from such medical procedures, namely the need of an invasive intervention. In fact, the above method can only be implemented by opening the body's region where the electrode is located, to allow an association between the reference and the counter electrode and the implant, with the consequent formation of an electric circuit.

Likewise, the Patent Application # US2017/0056536 discloses a method highly similar to the one revealed by the document US 2015/0076000, wherein a reference electrode, a counter electrode and a potentiostat are connected to a prosthesis. As described in one of the embodiments of the invention revealed therein, the prosthesis contains a conductor surface and a portion partially inserted inside the bone of a residual member. In this scenario, the potentiostat is connected to the conductor surface of the prosthesis, and the two other electrodes are connected to the potentiostat and to the external surface of the residual members. So, the potentiostat and the two electrodes form a three-spot electrochemical cell.

Nevertheless, such method also requires surgical intervention to allow the connection between the electrodes and a direct connection between the potentiostat and the prosthesis.

Thus, the state of art does not contemplate a device and/or a method that allows a full prevention and/or removal of the biofilm formed in the surface of metallic implants.

The present invention solves the concerns of the state of art by providing a device and a method that allow preventing biofilm formation at the surface of metallic implants and/or fully removing a previously formed biofilm layer. The invention uses a device that reaches its purposes on a non-invasive manner, without need of any surgical intervention.

### Objectives of the invention

A first objective of the present invention consists in providing a device able to generate an electric current in a conductor.

A second object of the present invention consists in providing a device able to generate an electric current in a conductor with portable dimensions.

A third objective of the present invention consists in providing a method to generate an electric current in a conductor.

### Brief Description of the Invention

The objectives of the present invention are achieved through the use of a device able to generate an electric current in a conductor, wherein the device comprises a magnetic field generator element, a control element, a support element and a energy source, wherein the magnetic field generator element and the control element are electrically associated to each other, wherein the magnetic field generator element is set to generate a variable magnetic field comprising at least one variable parameter, wherein the control element is set to control the at least one variable parameter and the support element is set to hold the energy source, the magnetic field generator element and the control element.

The objectives of the present invention are still achieved through the use of a method to generate electric current in a conductor through a device able to generate the electric current in the conductor, wherein the device comprises a magnetic field generator element, a control element, a support element and a energy source, wherein the magnetic field generator element and the control element are electrically associated to each other, wherein the magnetic field generator element is set to generate a variable magnetic field comprising at least one variable parameter, wherein the control element is set to control the at least one variable parameter and the support element is set to hold the energy source, the magnetic field generator element and the control element, and wherein the method is characterized by the fact that it comprises the following steps:
(a) establishing a value of at least one parameter of the desired electric current;
(b) establishing a time period for operating the device;
(c) adjusting the at least one variable parameter;
(d) generating a variable magnetic field;
(e) approaching the device to the conductor, and
(f) inducing the electric current in the conductor.

Additionally, the objectives of the present invention are achieved by using a method to remove biofilm from an internal conductor in a subject, through the use of a device able to generate an electric current in a conductor, wherein the device comprises a magnetic field generator element, a control element, a support element and a energy source, wherein the magnetic field generator element and the control element are electrically associated, wherein the magnetic field generator element is set to generate a variable magnetic field comprising at least one variable parameter, wherein the control element is set to control the at least one variable parameter and the support element is set to hold the energy source, the magnetic field generator element and the control element, wherein the method is characterized by the fact that it comprises the following steps:
(i) establishing the at least one variable parameter of the electric current as needed to the treatment of the biofilm inside the conductor;
(ii) establishing a time period during which said electric current shall be applied inside the conductor;
(iii) positioning the support element in the region of the subject's body where the conductor is located;
(iv) activating the device; and
(v) generating an electric current with the intensity as determined in the step i, for the period as determined in the step ii.

Further, the objectives of the present invention are also achieved through a method for treating a subject in need of removing biofilm from a metallic prosthesis implanted in his body, wherein the method comprises the steps of:
(a) establishing a value of at least one parameter of the desired electric current;
(b) establishing a time period for operating the device;
(c) adjusting the at least one variable parameter of the variable magnetic field;
(d) generating a variable magnetic field;
(e) approaching the device to the metallic implant, and
(f) inducing the electric current in the metallic implant.

### Summarized description of the drawings

The present invention shall be described in further details below, according to an exemplary embodiment represented by the drawings. The figures show:
Figure 1 - illustrates the device proposed herein and the components thereof.

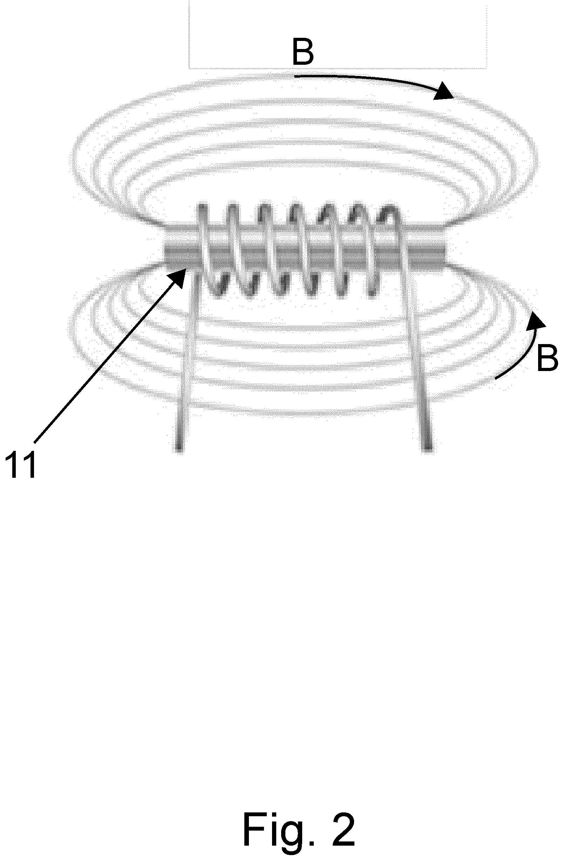

Figure 2 - illustrates the magnetic field generator element comprised by the device proposed herein.
Figure 3 - illustrates an embodiment of the present invention, wherein the device induces a current in a conductor through generation of an electromagnetic field.
Figure 4 - illustrates the device of the present invention, held in the support element.
Figure 5 - illustrates a possible embodiment of the magnetic field generator element, represented as a toroidal coil.

### Detailed Description of the Figures

As widely known from the state of art, the micro-organisms formed in a surface of implants from various materials, once inserted into the human body, may constitute the so-called biofilm.

As previously mentioned, such biofilms are structured communities of bacteria surrounded by polymeric matrices produced by themselves. Such polymeric substances provide protection to the bacteria against various types of possible aggressions, such as the use of any antibiotic or chemical agent to the elimination thereof.

In this scenario, biofilm is often formed in medical implants that hosted an infectious episode. Infection in medical implants, more specifically in prosthesis, affects approximately 2.4% of subjects using knee prosthesis, and up to 40% subjects with cardiac prosthesis. The most usual treatment involves surgery and invasive maneuvers, such as hospital admissions, rehabilitation periods and intensive treatment, or even removal and/or replacement of prosthesis.

Such procedures may result into further complications. Of note, any surgical procedure gives rise to the risk of other infections, as well as of possible clinical adverse conditions resulting from surgery.

As previously ascertained, such situation results from the fact that the biofilm formed in the surface of medical implants deprives antibiotic therapies of immediate effect, as biofilm protects bacteria against the effects from these products. Consequently, treatments only using antibiotics become inefficient, thus requiring surgical intervention.

However, a way to optimize the antibiotic activity consists in the use of electricity to fragilize the membranes of organisms comprised in the biofilm. The techniques usually encompass the use of invasive devices placed through an incision on the site of the implant. Then, the device is connected to the implant, and an electric current is generated for non-determined period. It is worth pointing out that the process described above is not used in the clinical praxis, not even on an experimental basis, as only invasive procedures are known.

Anyway, as previously mentioned, the need of performing an incision on the site of the implant is an undesired characterized that is circumvented by the present invention.

Of note, the present invention provides a device 10 and a method to generate an electric current in a conductor 15, in a non-invasive manner, to prevent and/or treat biofilm formation in such conductor 15, as addressed below.

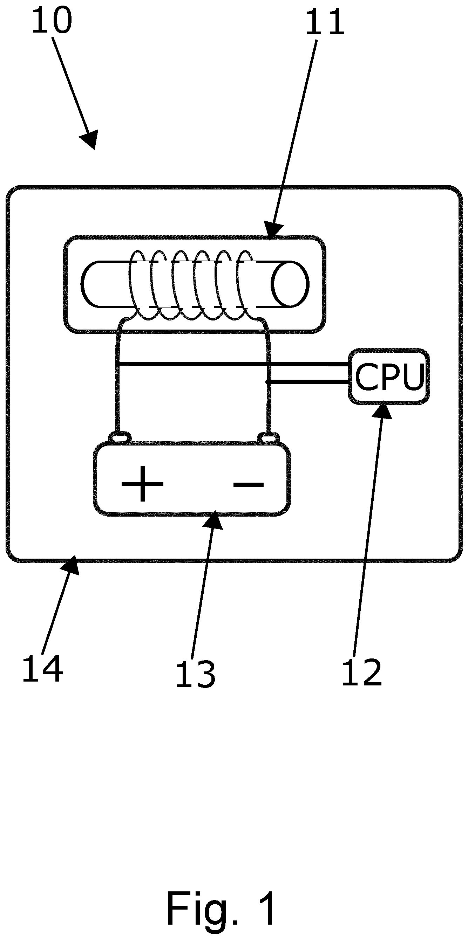

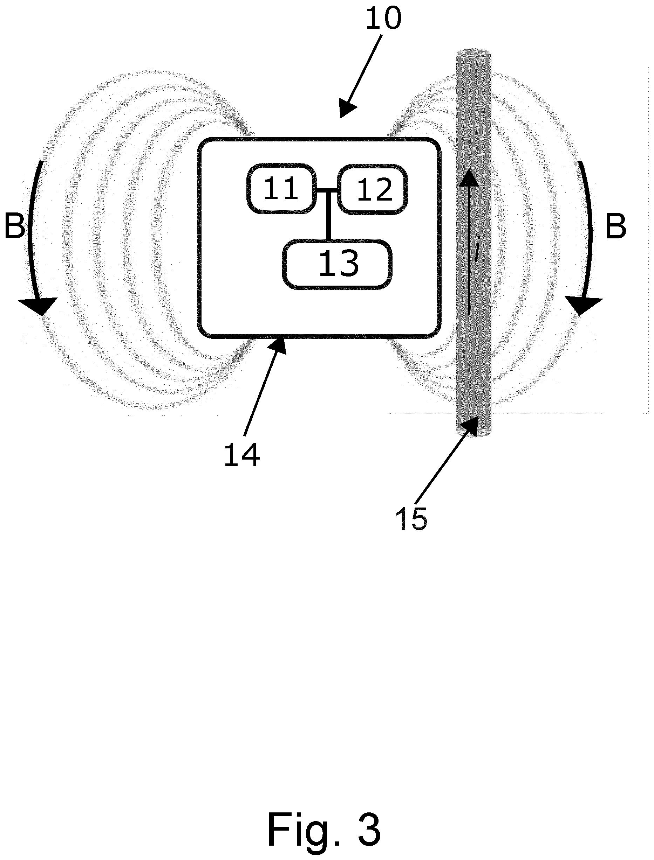

As observed in the figures 1 and 3, a device 10 is able to generate an electric current in a conductor 15. Such device comprises a magnetic field generator element 11 electrically associated to a control element 12, being both electrically associated to a energy source 13 and mechanically associated to a support element 14.

The magnetic field generator element 11 is set to generate a variable magnetic field B with, at least, a variable parameter, wherein such parameter can be any physical variable of the generated magnetic field. Only in an illustrative manner, without exhausting further possibilities, such parameter can be the intensity of the magnetic field, the polarity of the magnetic field and the position of the magnetic field, etc.

Such variable parameter is controlled by the control element 12 that, only in a preferential manner, can be a microprocessor with a software or any electronic control device previously known by the state of art.

Additionally, the magnetic field generator element 11 and the control element 12 are electrically fed by the energy source 13 that, only in an illustrative manner, can be a battery. However, such illustrative example should not be deemed as a limitation to the present invention, so that the energy source 13 can be any device that is able to electrically feed the device 10, or, in other words, to provide energy to operate the magnetic field generator element 11 and the control element 12.

Moreover, in the preferential embodiment described below, the energy source 13 must be always considered as electronically associated to the magnetic field generator element 11 and to the control element 12. So, whenever the magnetic field generator element 11 and the control element 12 are referred to herein, such reference shall also imply the presence of the energy source 13.

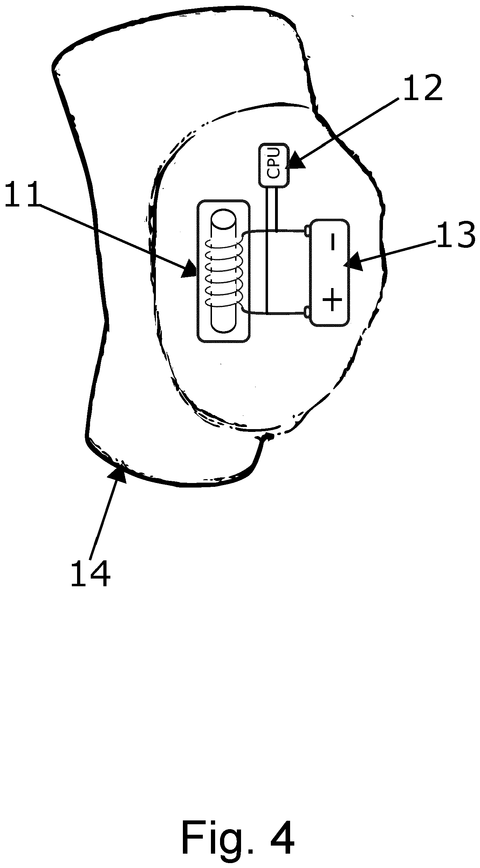

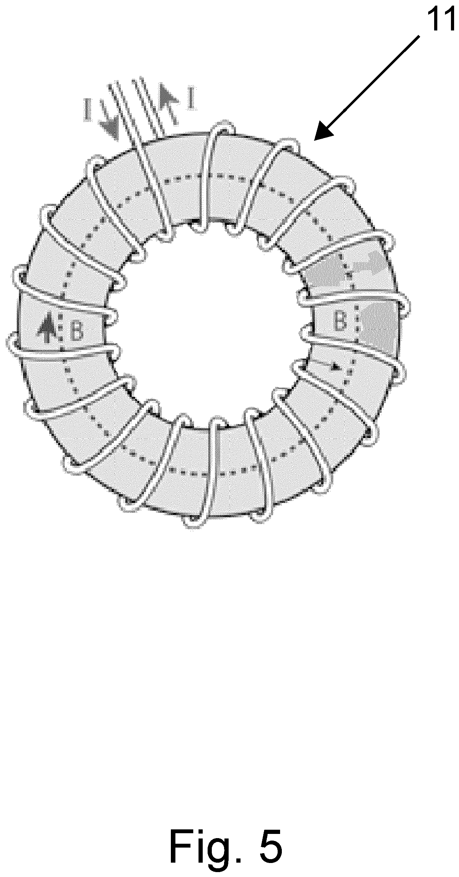

In reference to the figures 4 and 5, they only illustrate a preferential embodiment of the present invention, wherein the magnetic field generator element 11 is a toroidal coil and the energy source 13 is a battery. Said toroidal coil and said battery are set to be packed into the support element 14 that, only in an illustrative manner in this preferential embodiment, is an elbow pad or a knee pad.

Nevertheless, the above embodiment should not be understood as a limitation to the present invention, so that the magnetic field generator element 11 can be any device or apparatus able to generate a variable magnetic field, such as an electromagnet. Likewise, the support element 14 can be any element able to pack the magnetic field generator element 11, either inside or outside it.

Anyway, the above preferential embodiment intends to provide a device 10 that is able to generate an electric current in a conductor 15, wherein such device 10 is portable, with small dimensions as needed to possibly insert it in a knee pad or an elbow pad.

Additionally, the conductor 15 where the electric current shall be generated as a result from the device's operation is, only in a preferential embodiment, a metallic implant. More preferably, the metallic implant is a metallic prosthesis implanted inside the human body, in the region of the knee. Still more preferably, the metallic implant is a cobalt-chrome metallic knee prosthesis.

However, such features should not be understood as a limitation to the present invention, being, instead, mere illustrations of one of its possible embodiments. The conductor 15 can be a metallic conductor able to have a self-induced electric current. Moreover, the conductor 15 can be a metallic implant inserted at any region of the human body, possibly made of any metal alloy used by the state of art, such as titanium, steel, Teflon, etc.

In line with the information submitted so far, the device 10 shall, during its operation, induce an electric current in the relevant conductor 15.

This results from physical effects produced by several laws known by the state of art. Only in an illustrative manner, without any limitation, the Faraday's and Biot-Savart's Laws are related to a difference of potential and a current flow in an electric conductor, in presence of a magnetic field. Thus, the electric current is induced in the conductor 15 as a result from the variable magnetic field B generated by the magnetic field generator element 11, wherein at least a parameter of said variable magnetic field B is controlled by the control element 12.

Considering the merely preferential embodiment as previously described, wherein the conductor 15 is a chrome-cobalt metallic knee prosthesis, the magnetic field generator element 11 is a toroidal coil and the support element 14 is a knee pad, the magnetic field generator element 11 is placed inside the support element 14. Additionally, once the control element 12 is electrically associated to the magnetic field generator element 11, the control element 12 is also placed inside the support element 14. In other words, both control elements 12 and magnetic field generator 11 are inserted inside the support element 14.

Nevertheless, as previously mentioned, such arrangement should not be understood as a limitation to the present invention, but a mere illustration of a possible embodiment thereof. So, the magnetic field generator element 11 and the control element 12 can be arranged outside the support element 14, so that this latter is set to act as a support, for instance.

Further, as the magnetic field generator element 11 can be any element known by the state of art and able to generate a variable magnetic field, the dimensions of the support element 14 should not be specifically conditioned on the dimensions of the magnetic field generator element 11.

In other words, the support element 14 can be a knee pad as defined in the preferential embodiment of the present invention, as well as a metallic support, a box, a case, a cover, a coating, etc., or any other apparatus that is able to provide support to the magnetic field generator element 11.

Likewise, the magnetic field generator element 11 is only preferably a toroidal coil; however, such embodiment should not be deemed as a limitation to the present invention, as previously mentioned.

Likewise, in operation, the control element 12 shall control at least a variable parameter of the variable magnetic field B generated by the magnetic field generator element 11. So, thanks to the control of such variable parameter, it is possible to control the magnitude values of the electric current induced in the conductor 15 such as, for instance, the intensity i of the current, its polarity, frequency, modulation, etc. In other words, the control element 12 is also set to control the parameter values of the electric current induced in the conductor 15.

Moreover, the control element 12 is also set to control the period during which the magnetic field shall be generated by the magnetic field generator element 11, and consequently the period during which the electric current shall be induced in the conductor 15.

As to the -intensity i of the electric current induced in the conductor 15, only in a preferential manner, the electric current has a intensity i comprised within a range from 0.2 µA to 2 µA. Nevertheless, such range should not be understood as a limitation to the present invention, but as a merely preferential range as defined in relation to the preferential embodiment described herein. In other words, the electric current induced in the conductor 15 may comprise any desirable value consistent with the wished results in the period during which the device 10 shall be operated.

Further, and in line with the prior information, the present invention refers to a method to generate an electric current in a conductor 15, through a device 10 able to generate the electric current in this conductor 15.

Such method comprises the steps of:
(a) establishing a value of at least one parameters of the desired electric current;
(b) establishing a time period to the operation of the device 10;
(c) adjusting the at least one variable parameter;
(d) generating a variable magnetic field B;
(e) approaching the device 10 to the conductor 15; and
(f) inducing the electric current in the conductor 15.

Moreover, such method only represents a preferential embodiment of operation of the device 10, wherein the steps (a) and (b) are performed by the user. In other words, the desired values of the current and the period during which the current shall be induced in the conductor 15 are defined by the user, prior to the operation of the device 10.

So, once defined the values of the at least current parameter and period, the magnetic field variable parameter can be adjusted through the control element 12, so that the current showing the aforesaid characteristics is induced in the conductor 15.

Further, in the only preferential embodiment as previously described, the device 10 shall be placed inside the support element 14, so that the electric current shall be induced in the conductor 15, whenever the device 10 is put close to the conductor 15.

So, in a mere reference to the above described preferential embodiment, the electric current shall be induced when the device, arranged inside the knee pad, is put close to the knee prosthesis, or, in other words, as soon as the user wears the knee pad.

Additionally, and in line with the above information, the present invention refers to a method to remove biofilm from a conductor 15 in a subject, by using a device 10 that is able to generate an electric current with at least one variable parameter in such conductor 15.

Such method to remove biofilm from a conductor 14 in a subject comprises the steps of:
(i) establishing the at least one variable parameter of electric current as needed to treat the biofilm in the conductor 15;
(ii) establishing the time period during which said electric current shall be applied in the conductor 15;
(iii) placing the support element 14 at the region of the subject's body where the conductor 15 is located;
(iv) activating the device 10; and
(v) generating an electric current with the i intensity as established in the step i, for the period as established in the step ii.

Therefore, as previously mentioned, the activation of the device 10 shall result into the generation of a magnetic field B with at least a variable parameter that, on its turn, shall induce an electric current with at least a variable parameter in the conductor 15. Such electric current induction is, in an advantageous manner, responsible for the removal and/or treatment of the biofilm in the conductor 15.

Moreover, and only in a preferential embodiment of this method, the at least variable parameter of the electric current is at least one among: the i intensity of the electric current, the polarity of the electric current, the frequency of the electric current and the modulation of the electric current.

Additionally, in this preferential embodiment, the conductor 15 is a metallic implant, and still more preferably a metallic prosthesis.

Further, the present invention refers to a method to treat a subject in need of removing biofilm from a metallic prosthesis 15 implanted in his body, wherein the method comprises the steps of:
(a) establishing a value of at least one parameter of the desired electric current;
(b) establishing a time period to the operation of the device (10);
(c) adjusting the at least one variable parameter of the variable magnetic field B;
(d) generating the variable magnetic field B;
(e) approaching the device 10 to the metallic implant 15; and
(f) inducing the electric current in the metallic implant 15.

Once described an example of preferential embodiment, it must be understood that the scope of the present invention encompasses other possible variations, being exclusively limited by the content of the claims attached hereto, including further equivalents.

## Claims

1. Device (10) able to generate an electric current in a conductor (15), wherein the device (10) is **characterized by** the fact that it comprises a magnetic field generator element (11), a control element (12), a support element (14) and a energy source (13);
wherein the magnetic field generator element (11), the control element (12) and the energy source (13) are electrically associated;
wherein the magnetic field generator element (11) is set to generate a variable magnetic field (B);
wherein said variable magnetic field (B) comprises at least one variable parameter;
wherein the control element (12) is set to control the at least variable parameter; and
wherein the support element (14) is set to pack the energy source (13), the magnetic field generator element (11) and the control element (12).

2. Device (10) able to generate an electric current in a conductor (15) according to the claim 1, **characterized by** the fact that it has portable dimensions.

3. Device (10) able to generate an electric current in a conductor (15) according to the claim 1, **characterized by** the fact that the conductor (15) is an implant.

4. Device (10) able to generate an electric current in a conductor (15), according to the claim 3, **characterized by** the fact that the implant is a knee prosthesis.

5. Device (10) able to generate an electric current in a conductor (15) according to the claim 1, **characterized by** the fact that the conductor (15) is formed by at least one among: titanium, steel, Teflon and chrome-cobalt.

6. Device (10) able to generate an electric current in a conductor (15) according to the claim 1, **characterized by** the fact that the magnetic field generator element (11) is a toroidal coil.

7. Device (10) able to generate an electric current in a conductor (15) according to the claim 1, **characterized by** the fact that the control element (12) is a microprocessor.

8. Device (10) able to generate an electric current in a conductor (15) according to the claim 1, **characterized by** the fact that the at least one variable parameter is at least one physical variable of the variable magnetic field (B).

9. Device (10) able to generate an electric current in a conductor (15) according to the claim 5, **characterized by** the fact that the at least one variable parameter is at least one among: the intensity of the variable magnetic field (B), the polarity of the variable magnetic field (B), the frequency of the variable magnetic field (B) and the position of the variable magnetic field (B).

10. Device (10) able to generate an electric current in a conductor (15) according to the claim 1, **characterized by** the fact that the support element (14) is at least one between a knee pad and an elbow pad.

11. Device (10) able to generate an electric current in a conductor (15) according to the claim 1, **characterized by** the fact that the energy source (13) is set to electrically feed the components of the device (10).

12. Device (10) able to generate an electric current in a conductor (15) according to the claim 1, **characterized by** the fact that the energy source (13) is a battery.

13. Method to generate an electric current in a conductor (15) through a device (10) able to generate the electric current in the conductor (15),
wherein the device comprises a magnetic field generator element (11), a control element (12), a support element (14) and a energy source (13).
wherein the magnetic field generator element (11), the control element (12) and the energy source (13) are electrically associated;
wherein the magnetic field generator element (11) is set to generate a variable magnetic field (B);
wherein said variable magnetic field (B) comprises at least a variable parameter;
wherein the control element (12) is set to control the at least variable parameter, and
wherein the support element (14) is set to pack the energy source (13), the magnetic field generator element (11) and the control element (12),
and wherein the method is **characterized by** the fact that it comprises the following steps:
(a) establishing one value of at least one parameter of the desired electric current;
(b) establishing a time period for operating the device (10);
(c) adjusting the at least one variable parameter of the variable magnetic field (B);
(d) generating the variable magnetic field (B);
(e) approaching the device (10) to the conductor (15), and
(f) inducing the electric current in the metallic conductor (15).

14. Method to generate an electric current in a conductor (15) according to the claim 13, **characterized by** the fact that the steps (a) and (b) are performed by the user.

15. Method to generate an electric current in a conductor (15) according to the claim 13, **characterized by** the fact that the step (c) is performed by the control element (12).

16. Method to generate an electric current in a conductor (15) according to the claim 13, **characterized by** the fact that the step (d) is performed by the magnetic field generator element (11).

17. Method to generate an electric current in a conductor (15) according to the claim 13, **characterized by** the fact that the step (e) is performed by the user.

18. Method to generate an electric current in a conductor (15) according to the claim 13, **characterized by** the fact that the step (f) is performed by the device (10).

19. Method to generate an electric current in a conductor (15) according to the claim 13, **characterized by** the fact that the at least one parameter of electric current induced during the step (f) has its value as defined in the step (a).

20. Method to generate an electric current in a conductor (15) according to the claim 13, **characterized by** the fact that the at least one electric current parameter as defined in the step (a) corresponds to at least one among: the intensity of electric current (i), the polarity of the electric current, the frequency of the electric current and the modulation of the electric current.

21. Method to generate an electric current in a conductor (15) according to the claim 20, **characterized by** the fact that the intensity (i) of the electric current has a value comprised within a range from 0.2 µA to 2 µA.

22. Method to generate an electric current in a conductor (15) according to the claim 13, **characterized by** the fact that the step (f) is implemented during the period as defined in the step (b).

23. Method to remove biofilm from an internal conductor (15) in a subject by using a device (10) able to generate an electric current with at least one variable parameter in said conductor (15),
wherein the device (10) comprises a magnetic field generator element (11), a control element (12), a support element (14) and a energy source (13).
wherein the magnetic field generator element (11), the control element (12) and the energy source (13) are electrically associated,
wherein the magnetic field generator (11) is set to generate a variable magnetic field (B),
wherein said variable magnetic field (B) comprises at least a variable parameter;
wherein the control element (12) is set to pack the at least variable parameter of the electric current and of the variable magnetic field (B), and
wherein the support element (14) is set to pack the energy source (13), the magnetic field generator element (11) and the control element (12),
and wherein the method is **characterized by** the fact that it comprises the following steps:
(i) establishing the at least one variable parameter of the electric current as needed to the biofilm treatment in the conductor (15);
(ii) establishing the time period during which said electric current shall be applied to the conductor (15),
(iii) placing the support element (14) at the region of subject's body where the conductor (15) is located,
(iv) activating the device (10) and
(v) generating an electric current with an intensity (i) as established in the step i, during the period as determined in the step ii.

24. Method to remove biofilm from a conductor (15) according to the claim 23, **characterized by** the fact that the device (10) has portable dimensions.

25. Method to remove biofilm from a conductor (15) according to the claim 23, **characterized by** the fact that the conductor (15) is a metallic implant.

26. Method to remove biofilm from a conductor (15) according to the claim 24, **characterized by** the fact that the implant is a knee prosthesis.

27. Method to remove biofilm from a conductor (15) according to the claim 23, **characterized by** the fact that the step (iv) of activating the device (10) comprises generating an electromagnetic field (B) with at least a variable parameter.

28. Method to remove biofilm from a conductor (15) according to the claim 23, **characterized by** the fact that the electric current with intensity (i) as established in the step (i) is generated at the step (v) in accordance with the magnetic field (B) with at least one variable parameter generated in the step (iv).

29. Method to remove biofilm from a conductor (15) according to the claim 23, **characterized by** the fact that the at least one electric current parameter as defined in the step (i) corresponds to at least one among: intensity (i) of the electric current, polarity of the electric current, frequency of the electric current and modulation of the electric current.

30. Method to remove biofilm from a conductor (15) according to the claim 29, **characterized by** the fact that the intensity (i) of the electric current has a value comprised within a range from 0.2 µA to 2 µA.

31. Method for treating a subject in need of removing biofilm from a metallic prosthesis (15) implanted in his body, wherein the method is **characterized by** the fact that it comprises the steps of:
(a) establishing a value of at least one parameter of the desired electric current;
(b) establishing a time period to operate the device (10);
(c) adjusting the at least one variable parameter of the variable magnetic field (B);
(d) generating the variable magnetic field (B);
(e) approaching the device (10) to the metallic implant (15), and
(f) inducing the electric current in the metallic implant (15).

32. Method for treating a subject in need of removing biofilm from a metallic prosthesis (15) implanted inside his body according to the claim 31, **characterized by** the fact that the electric current induced in the step (f) has an intensity (i) comprised within a range from 0.2 µA to 2 µA.
